# EUROPEAN PATENT SPECIFICATION

(11) **EP 3 725 261 B1**
(45) Date of publication and mention of the grant of the patent: **12.01.2022**
(21) Application number: 19305487.1
(22) Date of filing: 15.04.2019
(51) Int. Cl.: A61C 8/02, A61C 13/00, A61F 2/28

(54) **METHOD AND APPARATUS FOR DYNAMICALLY ASSISTING A PRACTITIONER IN PREPARING A DENTAL BONE GRAFTING OPERATION**
VERFAHREN UND VORRICHTUNG ZUR DYNAMISCHEN UNTERSTÜTZUNG EINES PRAKTIKERS BEI DER VORBEREITUNG EINES ZAHNÄRZTLICHEN KNOCHENTRANSPLANTATIONSVORGANGS
PROCÉDÉ ET APPAREIL PERMETTANT D'AIDER UN PRATICIEN DYNAMIQUEMENT DANS LA PRÉPARATION D'UNE OPÉRATION DE GREFFE OSSEUSE DENTAIRE

(43) Date of publication of application: 21.10.2020
(73) Proprietor: Trophy, 77435 Marne La Vallée Cedex 2 (FR)
(72) Inventor: LAGARDERE, Aude, 77435 MARNE LA VALLEE CEDEX 2 (FR); BELCARI, Marianne, 77435 MARNE LA VALLEE CEDEX 2 (FR); ALRIC, Stephane, 77435 MARNE LA VALLEE CEDEX 2 (FR); SHELLARD, Edward R., 77435 MARNE LA VALLEE CEDEX 2 (FR)
(74) Representative: Santarelli

(56) References cited:
- EP-A1- 1 935 369
- WO-A1-2008/064840
- WO-A1-2015/132432
- US-A1- 2009 292 379
- US-B1- 8 483 863

## Description

### FIELD OF THE INVENTION

The present invention relates to the technical field of bone grafting and more specifically to a method and an apparatus for dynamically assisting a practitioner in preparing a dental bone grafting operation, for example in estimating characteristics of bone graft material needed during bone grafting planning.

### BACKGROUND OF THE INVENTION

Dental bone grafts are carried out to strengthen bone so that implants can be placed. To that end, one or more pieces of bone may be removed from another part of the body and used for the graft. Alternatively, bone grafting material or artificial bone material may be used.

In most cases, a first step in having dental bone grafting procedures completed involves some level of preparation. To that end, the jawbone of the patient is typically inspected using dental x-ray radiography. Some cases also require a CT-scan (i.e. computerized tomography scan) that may be considered as detailed 3-D x-ray radiography. As a result, it may be decided which type of bone grafting procedure is the most suited for the patient. Bone grafting techniques include, in particular, alveolar regeneration, the maxillary sinus lift, peri-implant regeneration, horizontal augmentation as well as periodontal regeneration. According to another technique, referred to as the apposition graft technique, one or more pieces of bone are grafted onto the jawbone. Such pieces of bone can be taken from another part of the body of the patient or can be pieces of artificial bone.

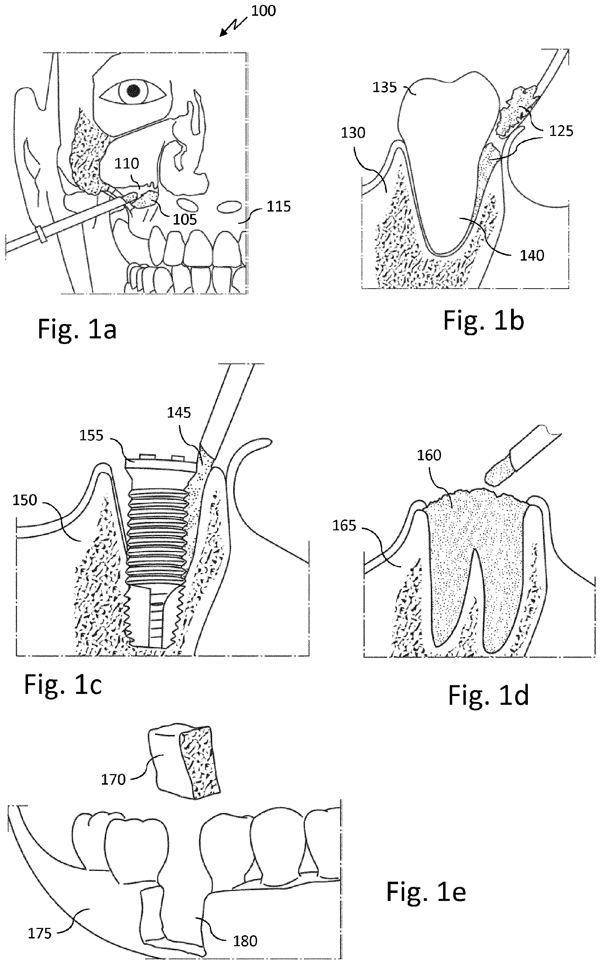

**Figures 1a to 1e** illustrate different techniques of bone grafting for jawbone.

A sinus lift is a surgical procedure that makes it possible to increase the thickness of the upper jaw by adding bone grafting material in the maxillary sinus. As illustrated in Figure 1a, bone grafting material 105 can be deposited in maxillary sinus 110 of upper jaw 115 of a patient 100 to increase thickness of upper jaw 115, making it possible to place an implant.

Periodontal regeneration makes it possible, for a patient, to keep healthy and functional teeth by maintaining the natural dental structure as much as possible. This may be obtained by regenerating bone and tissues, for example by introducing bone grafting material between the jawbone and a tooth root, as illustrated in Figure 1b. In the example illustrated in this Figure, bone grafting material 125 is deposited in a recess formed between jawbone 130 and root 140 of tooth 135.

As illustrated in Figure 1c, a similar technique can be used to correct an implant defect. As illustrated in Figure 1c, bone grafting material 145 can be deposited in a recess formed between jawbone 150 and implant 155.

Still based on a similar technique, alveolar regeneration aims at creating a bone base in place of a tooth root after the tooth has been removed, in order to enable placement of an implant. As illustrated in Figure 1d, bone grafting material 160 can be deposited in a cavity of jawbone 165, used to receive a tooth root, after the tooth has been removed.

According to another technique, bone grafting is conducted by bone apposition, by grafting one or several pieces of bone onto the jawbone. These pieces of bone can be removed from another part of the body or can be obtained from artificial bones. Figure 1e illustrates such a procedure according to which a piece of bone 170 is chosen as a function of a recess 180 formed in jawbone 175, where the bone graft is to be carried out, and put in place during surgery.

Other embodiments and examples of the prior art may be found in European Patent Application EP 1 935 369.

A dental surgeon determines a procedure to use and a source of bone grafting material depending on pathology characteristics and on the actual conditions. Next, the procedure is planned and prepared. During surgeon procedure, the dental surgeon fills in the target recess with the selected dental bone grafting material.

Except for the apposition graft technique, the dental bone grafting material is generally packaged in vials or syringes having a given capacity that may be expressed in grams or cubic centimetres (cc). Several vials or syringes may be needed for a single graft. Accordingly, the volume of the dental grafting material needed is preferably determined during preparation of the bone grafting so as to determine the corresponding number of vials or syringes.

While bone drafting techniques are commonly used and have proved to be efficient, there is a continuous need for improvement and for optimization, in particular for reasons of economy.

### SUMMARY OF THE INVENTION

The present invention has been devised to address one or more of the foregoing concerns.

In this context, there is provided a solution for dynamically estimating characteristics of bone graft material needed.

According to a first aspect of the invention, there is provided a method for dynamically assisting a practitioner in preparing a dental bone grafting operation, the method comprising:
- providing at least one image representing a 3D volume of at least a portion of a jaw member where bone grafting is to be carried out;
- adding to the at least one image a representation of a 3D virtual object representing dental bone graft material;
- estimating a value of at least one geometric characteristic relating to the bone graft material to be used, as a function of geometric characteristics of the represented 3D virtual object, the geometric characteristics of the represented 3D virtual object comprising at least one of a shape, a volume, and a size;
- enabling a user to modify the representation of the 3D virtual object; and
- upon modification of the representation of the 3D virtual object, updating the estimated value of the at least one geometric characteristic, according to the modification of the representation of the 3D virtual object,
the estimated value assisting the practitioner in determining the dental bone graft material needed.

According to the method of the invention, a practitioner may plan and prepare efficiently a bone grafting operation while optimizing the use of bone graft material in view of available bone graft material.

According to embodiments, the method further comprises selecting a type of grafting and determining the at least one geometric characteristic, the at least one geometric characteristic being determined as a function of the selected type of grafting.

According to embodiments, the value of the at least one geometric characteristic is further estimated as a function of the selected type of grafting.

According to embodiments, the method further comprises selecting at least one 3D base virtual object among a plurality of 3D base virtual objects, the plurality of 3D base virtual objects comprising 3D base virtual objects of different sizes and/or of different shapes, the 3D virtual object comprising the at least one selected 3D base virtual object.

According to embodiments, the at least one selected 3D base virtual object has a shape of an olive, a parallelepiped, a cone, or a combination thereof.

According to embodiments, the method further comprises selecting several 3D base virtual objects forming a set of 3D base virtual objects, the 3D virtual object resulting from the combination of the 3D base virtual objects of the set of 3D base virtual objects.

According to embodiments, the method further comprises homothetically adjusting the size of at least one of the at least one selected 3D base virtual object or homothetically adjusting the size of the 3D virtual object.

According to embodiments, the geometric characteristics of the represented 3D virtual object comprise at least one of a shape, a volume, and a size.

According to embodiments, the representation of the 3D virtual object comprises a plurality of points located on an external surface of the 3D virtual object, modifying the representation of the 3D virtual object comprising selecting one point of the plurality of points and moving the selected point, moving the selected point causing deforming the 3D virtual object accordingly.

According to embodiments, the selected point is selected in a 2D image representing a cross section of the 3D volume.

According to embodiments, the method further comprises selecting a standardized 3D virtual object among a plurality of standardized 3D virtual objects as a function of a size and a shape of the 3D virtual object, the selecting of a standardized 3D virtual object being repeated upon modification of the representation of the 3D virtual object.

According to embodiments, the method further comprises providing bone graft material corresponding to the selected standardized 3D virtual object and milling the provided bone graft material according to the 3D virtual object.

According to embodiments, the method further comprises generating a 3D model of the 3D virtual object, the generated 3D model making it possible 3D printing of a corresponding bone graft material or milling of bone graft material according to the 3D virtual object.

According to a second aspect of the invention, there is provided a device for dynamically estimating characteristics of dental bone graft material needed, the device comprising a microprocessor configured for carrying out each of the steps of the method described above.

The second aspect of the present invention has advantages similar to the first above-mentioned aspect.

At least parts of the methods according to the invention may be computer implemented. Accordingly, the present invention may take the form of an entirely hardware embodiment, an entirely software embodiment (including firmware, resident software, micro-code, etc.) or an embodiment combining software and hardware aspects that may all generally be referred to herein as a "circuit", "module" or "system". Furthermore, the present invention may take the form of a computer program product embodied in any tangible medium of expression having computer usable program code embodied in the medium.

Since the present invention can be implemented in software, the present invention can be embodied as computer readable code for provision to a programmable apparatus on any suitable carrier medium. A tangible carrier medium may comprise a storage medium such as a floppy disk, a CD-ROM, a hard disk drive, a magnetic tape device or a solid state memory device and the like. A transient carrier medium may include a signal such as an electrical signal, an electronic signal, an optical signal, an acoustic signal, a magnetic signal or an electromagnetic signal, e.g. a microwave or RF signal.

### BRIEF DESCRIPTION OF THE DRAWINGS

Other features and advantages of the invention will become apparent from the following description of non-limiting exemplary embodiments, with reference to the appended drawings, in which:
**Figures 1a to 1e** illustrate different techniques of bone grafting for jawbone;
**Figure 2** illustrates an example of steps of a method according to embodiments of the invention;
**Figure 3** illustrates a first example of use of a method according to embodiments of the invention such as the one described by reference to Figure 2;
**Figure 4**, comprising Figures 4a to 4e, illustrates a second example of use of a method according to embodiments of the invention such as the one described by reference to Figure 2; and
**Figure 5** is a schematic block diagram of a computing device for implementing embodiments of the invention.

### DETAILED DESCRIPTION OF EMBODIMENTS OF THE INVENTION

The inventors have observed that the bone grafting material used for a graft operation has a significant cost depending on the amount of graft material needed and potentially on the graft shape, that should be optimized.

According to embodiments, the characteristics of the bone grafting material needed is determined dynamically by a user or practitioner when preparing a graft operation, based on a 3D virtual object representing a region of interest characterizing a graft location in images of a 3D volume, for example in a CBCT image (i.e. a cone beam computerized tomography image), of at least a portion of jaw members.

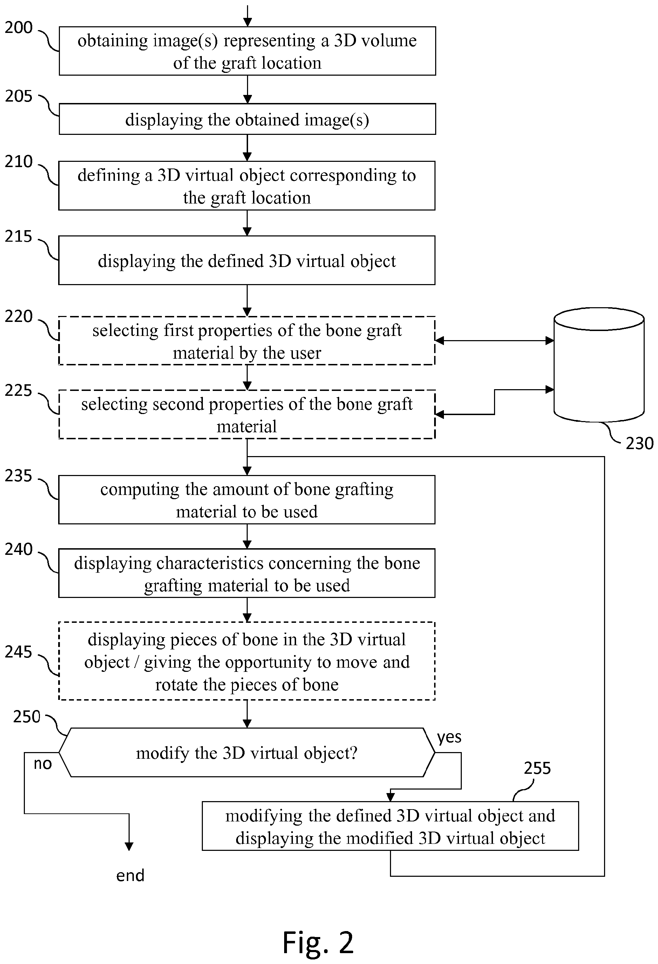

**Figure 2** illustrates an example of steps of a method according to embodiments of the invention.

As illustrated, a first step is directed to obtaining one or more images, for example CBCT images or images obtained using the ultra-sound technology, representing a 3D volume of the location where the graft is to be done and of the surrounding area (step 200).

According to embodiments, at least one 3D volume image is obtained. It may result from the processing of many 2D images of the same area taken from different points of view, for example several hundreds of images. Such a 3D volume image may have been previously computed or not. Alternatively, it is computed from obtained 2D images. 3D volume images may be obtained automatically or on practitioner request.

According to other embodiments, several images representing different cross section views of the 3D volume of the location where the graft is to be done are obtained.

Next, the obtained image(s) representing a 3D volume are displayed (step 205). To that end, a 3D image viewer such as the Carestream 3D Viewer software application, known as CS 3D imaging software, may be used (Carestream is a trademark).

Next, a 3D virtual object representing a region of interest is defined and then displayed (steps 210 and 215). It corresponds to the graft location, that is to say to an approximate of the volume that is to be filled in by bone grafting material (taking into account parameters such as an expansion / contraction coefficient or other physical/chemical properties). Such a volume may be geometrically defined or may be defined by a set of voxels.

Several methods can be used to define the 3D virtual object corresponding to the graft location.

According to a particular embodiment, a 3D virtual object having a default shape and a default size is selected. It may correspond to the most common shape and to a mean size of dental bone graft. In such a case, the practitioner may just indicate the location where the 3D virtual object is to be located in the displayed images, for example by identifying the center of the 3D virtual object.

According to other embodiments, a shape may be chosen in a library comprising different shapes. Such different shapes may be presented on a graphical user interface so that a practitioner may select one of them. Likewise, a size may be chosen by the practitioner in a set of predefined sizes, for example a set of normalized sizes. Still according to other embodiments, a 3D virtual object may be automatically selected from a library from instructions given by the practitioner, for example by entering, on displayed images, a plurality of points forming the boundary of the 3D virtual object. A 3D virtual object may also be defined automatically as a result of image analysis, for example by image segmentation and/or texture analysis.

The 3D virtual object may also be defined as a function of one or several seeds drawn by a practitioner on images, from which complex 3D surfaces can be automatically created. As a further example, the 3D virtual object may be defined by defining contours on 2D sections such as sections known as multiplanar reformatted (MPR), from which a 3D volume may be constructed.

As described above, once the 3D virtual object has been defined, it is displayed on the displayed images. For the sake of illustration, the 3D virtual object may be represented by drawing its contour, for example with a particular colour.

At this stage, the practitioner may chose a type of grafting, for example apposition or another type of grafting.

Next (or before or in parallel), first properties of the bone grafting material to be used may be selected (step 220). Such properties may depend on whether the bone grafting material comprises one or more pieces of bone or bone grafting material provided in vials or syringes such as a powder material, i.e. may depend on the type of grafting. If the bone grafting material is one or more pieces of bone, such properties may comprise a type of bone to be used, standardized shapes, and/or standardized sizes. Alternatively, if the bone grafting material is material provided in vials or syringes such as a powder material, such properties may comprise a type of material, a type of packaging (e.g. vial or syringe), and the standardized amount of material packaged.

From the selected first properties, second properties may be automatically obtained (step 225). For example, an expansion / contraction coefficient and/or a density coefficient may be obtained automatically from the selected type of grafting material. According to particular embodiments, the second properties may comprise a type of packaging (e.g. vial or syringe) and/or the amount of material packaged that are determined as a function of a type of material (it being noted that there may exist several doses, that is to say several possible amounts of material for a given packaging).

Still according to embodiments, some of the properties are selected by a practitioner from a library (denoted 230 in Figure 2) that may be identified by the practitioner or that can be automatically identified and other properties are obtained from this library or from another library as a function of the selected properties.

Next, the amount of bone grafting material is computed (step 235). The amount of bone grafting material may be determined, in particular, by a volume or a weight. According to embodiments, the volume of bone grafting material corresponds, before taking into account an expansion factor, to the volume of the 3D virtual object.

During the computation of the amount of bone grafting material, other characteristics may be determined, for example the shape of the 3D virtual object, making it possible to select one or more standardized pieces of bone to be grafted (if the bone grafting material is one or more pieces of bone). In such a case, the amount of bone grafting material or a size may be used in conjunction with the shape of the 3D virtual object to select one or more pieces of bone, standardized or not, from a library.

Alternatively, if the bone grafting material is material provided in vials or syringes, the computed amount of bone grafting material may be used to determine a number of vials or syringes, depending on the properties of the selected bone grafting material, its packaging, and the computed amount of bone grafting material.

In response to the computation of the amount of bone grafting material, this amount of bone grafting material as well as, optionally, the shape of the piece of bone to be used or the number of vials or syringes to be used are displayed (step 240).

The cost of the bone grafting material is preferably also computed and displayed. The bone grafting material may also be ordered.

According to particular embodiments, if the bone grafting material is one or more pieces of bone, one or more standardized pieces of bone selected as a function of the 3D virtual object may be displayed so that the practitioner may compare the shape of this or these pieces of bone with the shape of the 3D virtual object (step 245). Still according to particular embodiments, the practitioner may move or rotate each of the pieces of bone to verify that they match with the 3D virtual object.

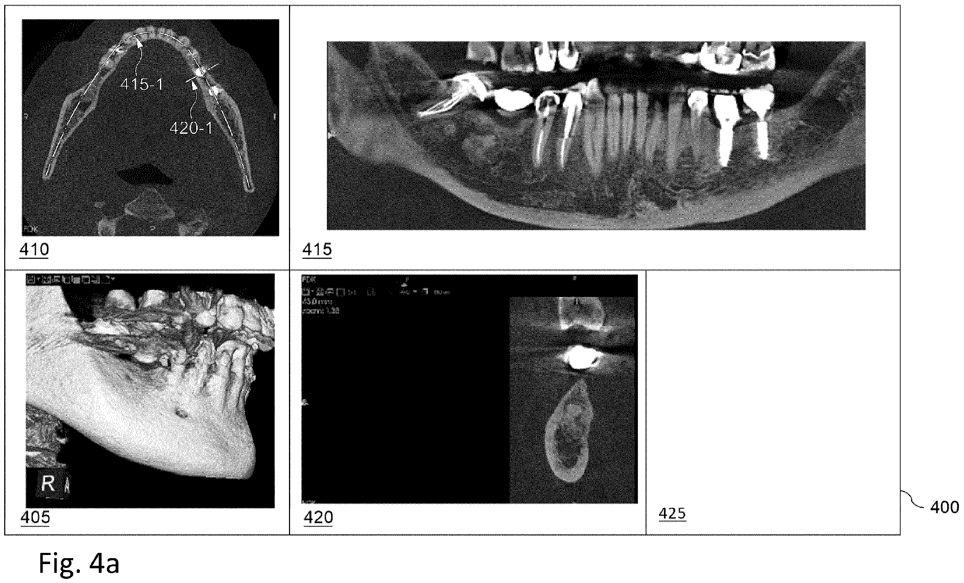

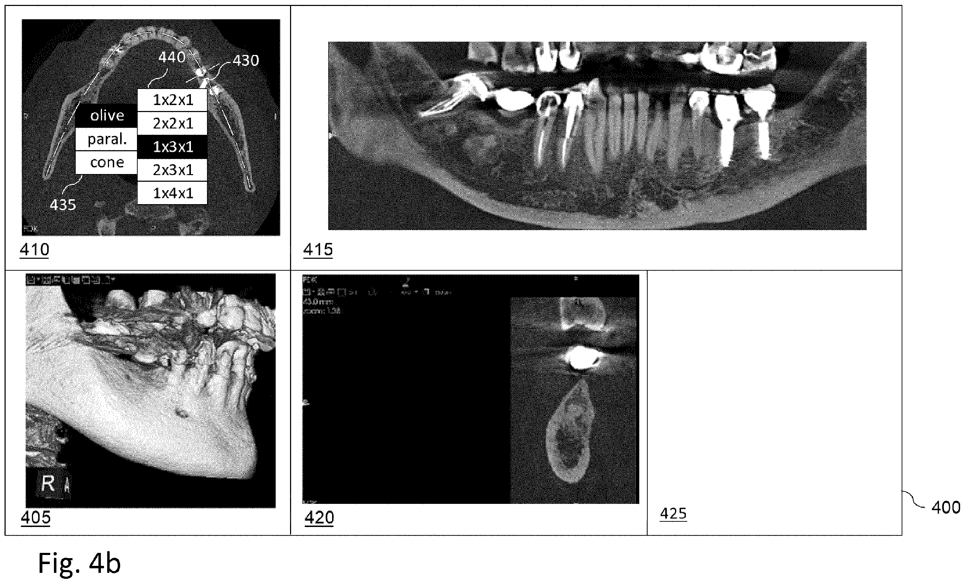

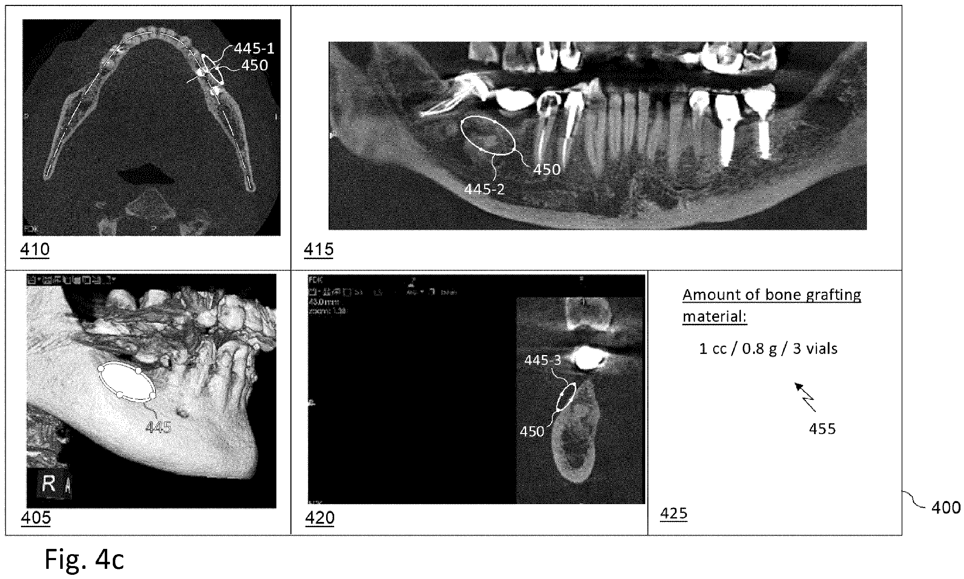

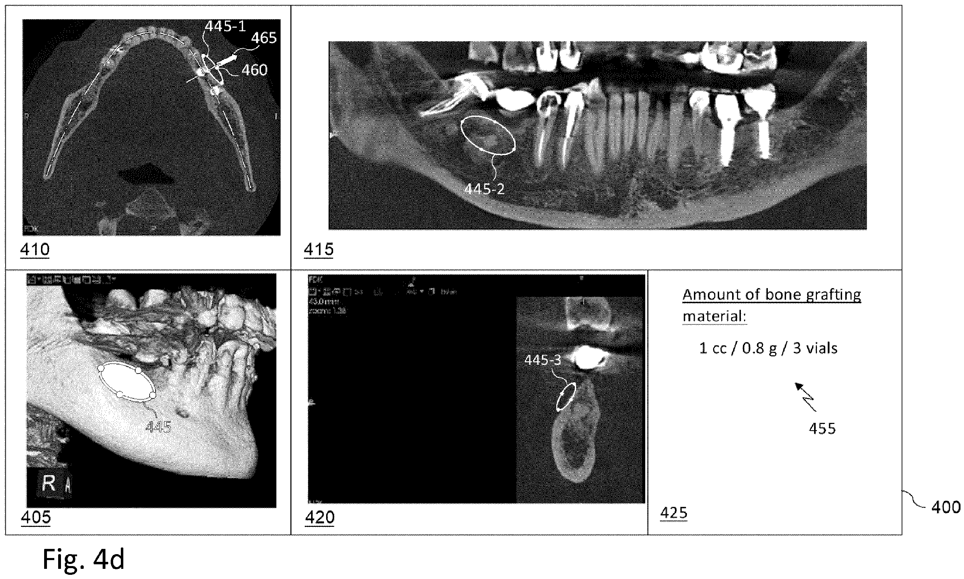

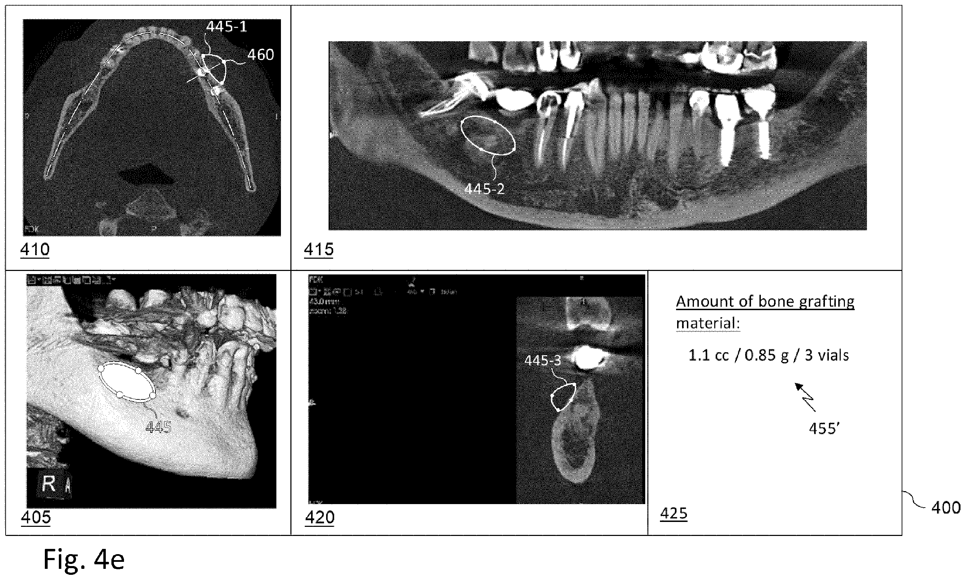

Next, an opportunity is given to the practitioner to modify the 3D virtual object corresponding to the graft location (step 250). For the sake of illustration, the opportunity may be given to the practitioner to deform the 3D virtual object, for example to increase or to reduce its size by moving a portion of its surface. As described with reference with Figure 4, this can be done by selecting a point on the outer surface of the 3D virtual object, for example a point represented in a cross section view of the 3D volume of the grafting location, and moving the selected point. The outer surface of the 3D virtual object is deformed accordingly.

If the practitioner needs to modify the 3D virtual object, for example to optimize the use of bone grafting material, he/she modifies it (step 255). Further to modifying the 3D virtual object and displaying the modified 3D virtual object, the algorithm loops on step 235 so that the amount of bone grafting material corresponding to the modified 3D virtual object is computed and displayed.

On the contrary, if the practitioner does not need to modify the 3D virtual object, the algorithm ends.

In case of apposition grafting, i.e. when the 3D virtual object represents one or more pieces of bone, a 3D model may be generated for preparing the bone grafting material, that is to say the one or more pieces of bone. Such a 3D model may be used for 3D printing the piece(s) of bone or for milling the piece(s) of bone from standardized piece(s) of bone. The standardized piece(s) of bone are advantageously selected from a library of standardized pieces of bone, when the corresponding 3D virtual object is created or modified, in such a way that the selected standardized piece(s) of bone are the closest to the 3D virtual model and greater than the latter.

According to particular embodiments, if the bone grafting material is material provided in vials or syringes and if there exist several doses for the bone grafting material that has been selected, the conditioning to be used (that is generally normalized) may be chosen so as to optimize costs.

Still according to particular embodiments, if the bone grafting material is based on pieces of bone, the choice of the pieces of bone to be used is determined automatically or by the practitioner, in particular as a function of their shape, so that the combination of the chosen pieces of bone matches the defined 3D virtual object and so as to optimize the cost of the chosen pieces of bone. For the sake of illustration, if the shape of the bone grafting material is an "L", the pieces of bone used for making the bone grafting material may be the two branches of the "L". Each of these pieces of bone may be represented as a 3D base virtual object. By using a small number of elementary shapes such as an olive, a parallelepiped, and a cone, of different sizes, complex 3D virtual object can be made.

Displaying characteristics concerning the bone grafting material needed and giving the opportunity to the practitioner to modify the 3D virtual object corresponding to the graft location enable the practitioner to optimize the graft parameters.

According to embodiments, the size of the displayed 3D virtual object may be homothetically adjusted or modified (i.e. the size of the 3D virtual object is modified but its shape and proportions are not modified). Similarly, the practitioner may adjust or modify the 3D base virtual object(s).

It is to be noted that several 3D virtual objects may be created and displayed on the same images. These 3D virtual objects may be adjacent or not.

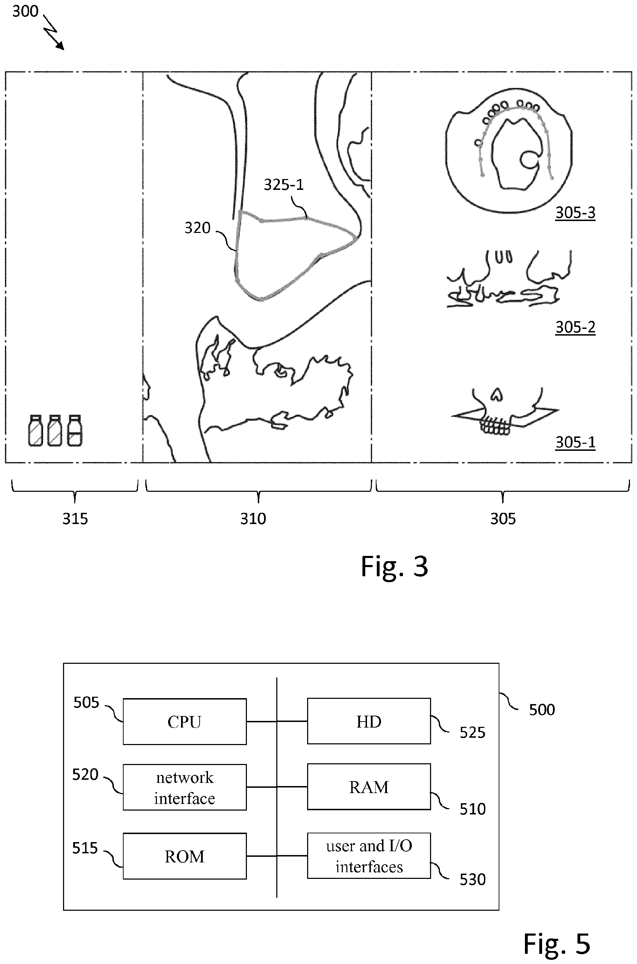

**Figure 3** illustrates a first example of use of a method according to embodiments of the invention such as the one described by reference to Figure 2. More specifically, Figure 3 schematically represents a screen shot of a graphical user interface of a computer application implementing such a method.

As illustrated, the graphical user interface comprises several areas for displaying information relative to the patient's dental structure, to the graft location, and to the needed bone grafting material, and for making it possible for a practitioner to define and modify a 3D virtual object representing the bone grafting material.

For the sake of illustration, the graphical user interface 300 comprises three main areas denoted 305, 310, and 315.

Area 305 provides different views of the patient dental structure, for example a coloured 3D perspective view (305-1), a global horizontal view of the jaw (305-3), and a detail view (305-2) that can be adjusted easily by the practitioner (who is typically a dentist or a dental surgeon).

Area 310 illustrates a 2D section of the graft location and of the surrounding dental structure, wherein the contour of the 3D virtual object corresponding to the graft location is represented (reference 320). According to the illustrated example, the 3D virtual object is defined by a contour, in a plurality of parallel 2D sections, each contour being defined by a set of points (e.g. point 325-1) that are joined, defining a 3D surface in connection with the upper and the lower 2D sections.

It is to be noted that the view illustrated in area 310 may depend on how the 3D virtual object can be defined and/or modified by the practitioner.

Area 315 represents the characteristics relating to the amount of bone grafting material and optionally the shape of the pieces of bone to be used or the number of vials or syringes to be used. According to embodiments, it comprises the cost of the bone grafting material. Still according to embodiments, it comprises the conditioning of the bone grafting material or the shape of the pieces of bone as determined for optimizing the costs. Other characteristics may be displayed.

It is to be understood that the graphical user interface is not limited to these three areas. It may comprise more views or only the areas 310 and 315.

According to embodiments, the number and the nature of the views displayed on the graphical user interface can be configured by the practitioner.

**Figure 4**, comprising Figures 4a to 4e, illustrates a second example of use of a method according to embodiments of the invention such as the one described by reference to Figure 2. More specifically, Figures 4a to 4e schematically represent screen shots of a graphical user interface of a computer application implementing such a method, when creating and modifying a 3D virtual object representing bone grafting material.

As illustrated in Figure 4a, the graphical user interface comprises several areas for displaying information relative to the patient's dental structure, to the graft location, and to the needed bone grafting material, and for making it possible for the practitioner to define and modify a 3D virtual object representing the bone grafting material.

For the sake of illustration, the graphical user interface 400 comprises five main areas denoted 405, 410, 415, 420, and 425.

Still for the sake of illustration, area 405 represents a 3D view of a portion of a jaw member. According to embodiments, the graphical user interface makes it possible to rotate the represented portion of the jaw member and/or to zoom/shrink into it. This can be done, for example, according to multi-touch gestures enabling a touchscreen or a trackpad to interact with the software displaying the portion of the jaw member.

As illustrated, area 410 represents a cross section view of the jaw member portion displayed in area 405, according to a horizontal plan of view whose height may be modified by the practitioner through the graphical user interface. According to this example, area 410 further illustrates two plan curves, denoted 415-1 and 420-1, that defines two vertical cross section plans. Curve 415-1 is defined as the median (in the horizontal plan) of the upper or lower jawbone and curve 420-1 is a segment approximately perpendicular to curve 415-1 at a location defined by the practitioner.

Area 415 represents a cross section view of the jaw member portion displayed in area 405, according to the vertical plan of view defined by curve 415-1 in area 410. Likewise, area 420 represents a cross section view of the jaw member portion displayed in area 405, according to the vertical plan of view defined by curve 420-1 in area 410.

Area 425 is used to display items of information regarding bone grafting material.

As apparent from Figure 4a, the graphical user interface comprises many items of information and many commands for manipulating and processing the representations of the jaw member.

Naturally, other dispositions may be used.

As illustrated in Figure 4b, a practitioner may select a location, for example using a pointer such as a mouse in one of the images representing the portion of the jaw member, for example location 430 in the cross section view of area 410. Next, by using a specific command, the practitioner may create a 3D virtual object, for example by selecting a shape and a size in dedicated menus 435 and 440. Such menus may appear, for example, using the right click of a mouse.

According to other embodiments, a 3D virtual object having a default shape and a default size may be created when the practitioner selects a location and requests the creation of a 3D virtual object. Still according to other embodiments, the practitioner may select two or more location for entering a size of the 3D virtual object to be created. In such a case, the practitioner may select the shape of the 3D virtual object to create or a default shape may be used.

Still according to particular embodiment, a 3D virtual object may result from combining several 3D base virtual objects. Such 3D base virtual objects may be selected as described above regarding a 3D virtual object. The resulting 3D virtual object may correspond to the outer surface forms by the combination of the 3D base virtual objects.

As illustrated in Figure 4c, creation of a 3D virtual object results in adding its representation in the displayed images representing the portion of the jaw member, at the corresponding location and at the right scale, according to the selected shape and size.

According to the given example and as displayed on the displayed images with references 445, 445-1, 445-2, and 445-3, the created 3D virtual object has a shape of an olive. Its location is the one defined by the practitioner during creation. For the sake of illustration, it is defined by a set of points, generically referred to as 450, that belong to the outer surface of the 3D virtual object and to a tangent plan. Points may be added or removed for adapting the shape of the 3D virtual object to the planned graft.

As described with reference to Figure 2, an amount of bone grafting material is determined after a 3D virtual object is created or modified, preferably taking into account parameters such as an expansion / contraction coefficient or other physical/chemical properties. Such parameters may derived from a choice of the practitioner (e.g. a type of bone grafting material) and technical specification stored in a database. Accordingly, after 3D virtual object 445 is created, the corresponding amount of bone grafting material is determined and items of information representing this amount is computed, as illustrated with reference 455. Such an amount may be expressed in different units such as in cubic centimetres, grams, or number of vials or syringes.

As illustrated in Figure 4d, the practitioner may select one point of the set of points 450, for example point denoted 460 in area 410, and move it, for example along direction 465.

This results in deforming 3D virtual object 445, as illustrated in Figure 4e, in particular with reference 445-1 in area 410, and in redetermining the corresponding amount of bone grafting material, as illustrated with reference 455'. It is observed here that the amount of bone grafting material, expressed in number of vials does not change (contrary to the amount of bone grafting material expressed in cubic centimetres or in grams) since (for the sake of illustration) the vials where not fully used in the previous configuration.

When the bone grafting material is a piece of bone (apposition graft), a standardized piece of bone may be automatically selected in a library containing standardized pieces of bone, of different shapes and of different sizes. The selected piece of bone is preferably the one that is the closed to the 3D virtual object and that size is greater than the one of the 3D virtual object, making it possible milling the selected standardized piece of bone to obtain the needed piece of bone. Such a standardized piece of bone is selected each time a 3D virtual object is created or modified.

**Figure 5** is a schematic block diagram of a computing device for implementation of one or more embodiments of the invention, in particular for carrying out the steps or parts of the steps described by reference to Figure 2 and the graphical user interface illustrated in Figures 3 and 4.

Computing device 500 comprises a communication bus connected to:
- a central processing unit 505, such as a microprocessor, denoted CPU;
- a random access memory 510, denoted RAM, for storing the executable code of the method of embodiments of the invention as well as the registers adapted to record variables and parameters necessary for implementing the method for dynamically estimating bone graft material according to embodiments of the invention, the memory capacity of which can be expanded by an optional RAM connected to an expansion port for example;
- a read only memory 515, denoted ROM, for storing computer programs for implementing embodiments of the invention; and
- a user interface and/or an input/output interface 530 which can be used for receiving inputs from a user, displaying information to a user, and/or receiving/sending data from/to external devices.

Optionally, the communication bus of computing device 500 may be connected to:
- a hard disk 525 denoted HD used as a mass storage device; and/or
- a network interface 520 typically connected to a communication network over which digital data can be transmitted or received. The network interface 520 can be a single network interface, or composed of a set of different network interfaces (for instance wired and wireless interfaces, or different kinds of wired or wireless interfaces). Data packets are written to the network interface for transmission or are read from the network interface for reception under the control of the software application running in the CPU 505.

The executable code may be stored either in read only memory 515, on hard disk 525 or on a removable digital medium such as for example a disk. According to a variant, the executable code of the programs can be received by means of a communication network, via the network interface 520, in order to be stored in one of the storage means of the communication device 500, such as hard disk 525, before being executed.

Central processing unit 505 is adapted to control and direct the execution of the instructions or portions of software code of the program or programs according to embodiments of the invention, the instructions being stored in one of the aforementioned storage means. After powering on, CPU 505 is capable of executing instructions from main RAM memory 510 relating to a software application after those instructions have been loaded from ROM 515 or from hard-disk 525 for example. Such a software application, when executed by CPU 505, causes the steps of the algorithms herein disclosed to be performed.

Any step of the algorithm herein disclosed may be implemented in software by execution of a set of instructions or program by a programmable computing machine, such as a PC ("Personal Computer"), a DSP ("Digital Signal Processor") or a microcontroller; or else implemented in hardware by a machine or a dedicated component, such as an FPGA ("Field-Programmable Gate Array") or an ASIC ("Application-Specific Integrated Circuit").

While the invention has been illustrated and described in detail in the drawings and foregoing description, such illustration and description are to be considered illustrative or exemplary and not restrictive, the invention being not restricted to the disclosed embodiment.

In the claims, the word "comprising" does not exclude other elements or steps, and the indefinite article "a" or "an" does not exclude a plurality. A single processor or other unit may fulfil the functions of several items recited in the claims. Any reference signs in the claims should not be construed as limiting the scope of the invention.

## Claims

1. A computer method for dynamically assisting a practitioner in preparing a dental bone grafting operation, the method comprising:
- providing at least one image representing a 3D volume of at least a portion of a jaw member where bone grafting is to be carried out;
- adding to the at least one image a representation of a 3D virtual object representing dental bone graft material (110, 125, 145, 160, 170);
- estimating a value of at least one geometric characteristic relating to the bone graft material to be used, as a function of geometric characteristics of the represented 3D virtual object, the geometric characteristics of the represented 3D virtual object comprising at least one of a shape, a volume, and a size;
- enabling a user to modify the representation of the 3D virtual object; and
- upon modification of the representation of the 3D virtual object, updating the estimated value of the at least one geometric characteristic, according to the modification of the representation of the 3D virtual object, the estimated value assisting the practitioner in determining the dental bone graft material (110, 125, 145, 160, 170) needed.

2. The method of claim 1, further comprising selecting a type of grafting, by a practitioner, and determining the at least one geometric characteristic, the at least one geometric characteristic being determined as a function of the selected type of grafting.

3. The method of claim 2, wherein the value of the at least one geometric characteristic is further estimated as a function of the selected type of grafting.

4. The method of any one of claims 1 to 3, further comprising selecting at least one 3D base virtual object among a plurality of 3D base virtual objects, the plurality of 3D base virtual objects comprising 3D base virtual objects of different sizes and/or of different shapes, the 3D virtual object comprising the at least one selected 3D base virtual object.

5. The method of claim 4, wherein the at least one selected 3D base virtual object has a shape of an olive, a parallelepiped, a cone, or a combination thereof.

6. The method of claim 4 or claim 5, comprising selecting several 3D base virtual objects forming a set of 3D base virtual objects, the 3D virtual object resulting from the combination of the 3D base virtual objects of the set of 3D base virtual objects.

7. The method of any one of claims 4 to 6, further comprising homothetically adjusting the size of at least one of the at least one selected 3D base virtual object or homothetically adjusting the size of the 3D virtual object.

8. The method of any one of claims 1 to 7, wherein the representation of the 3D virtual object comprises a plurality of points located on an external surface of the 3D virtual object, modifying the representation of the 3D virtual object comprising selecting one point of the plurality of points and moving the selected point, moving the selected point causing deforming the 3D virtual object accordingly.

9. The method of claim 8, wherein the selected point is selected in a 2D image representing a cross section of the 3D volume.

10. The method of any one of claims 1 to 9, further comprising selecting a standardized 3D virtual object among a plurality of standardized 3D virtual objects as a function of a size and a shape of the 3D virtual object, the selecting of a standardized 3D virtual object being repeated upon modification of the representation of the 3D virtual object.

11. The method of claim 10, further comprising providing bone graft material corresponding to the selected standardized 3D virtual object and milling the provided bone graft material according to the 3D virtual object.

12. The method of any one of claims 1 to 10, further comprising generating a 3D model of the 3D virtual object, the generated 3D model making it possible 3D printing of a corresponding bone graft material or milling of bone graft material according to the 3D virtual object.

13. A computer program product for a programmable apparatus, the computer program product comprising instructions for carrying out each step of the method according to any one of claims 1 to 12 when the program is loaded and executed by a programmable apparatus.

14. A device for dynamically estimating characteristics of dental bone graft material needed, the device comprising a microprocessor configured for carrying out each of the steps of the method according to any one of claims 1 to 12.

## Patentansprüche

1. Computerverfahren zur dynamischen Unterstützung eines Arztes bei der Vorbereitung einer dentalen Knochentransplantationsoperation, wobei das Verfahren umfasst:
- Bereitstellen mindestens eines Bildes, das ein 3D-Volumen mindestens eines Teils eines Kieferelements darstellt, an dem eine Knochentransplantation durchgeführt werden soll;
- Hinzufügen einer Darstellung eines virtuellen 3D-Objekts, das dentales Knochentransplantatmaterial (110, 125, 145, 160, 170) darstellt, zu dem mindestens einen Bild;
- Schätzen eines Wertes mindestens eines geometrischen Merkmals, das sich auf das zu verwendende Knochentransplantatmaterial bezieht, als eine Funktion von geometrischen Merkmalen des dargestellten virtuellen 3D-Objekts, wobei die geometrischen Merkmale des dargestellten virtuellen 3D-Objekts mindestens eines von einer Form, einem Volumen und einer Größe umfassen;
- Ermöglichen, dass ein Benutzer die Darstellung des virtuellen 3D-Objekts ändern kann; und
- bei Änderung der Darstellung des virtuellen 3D-Objekts Aktualisieren des geschätzten Werts des mindestens einen geometrischen Merkmals entsprechend der Änderung der Darstellung des virtuellen 3D-Objekts,
wobei der geschätzte Wert den Arzt bei einer Bestimmung des benötigten Knochentransplantatmaterials (110, 125, 145, 160, 170) unterstützt.

2. Verfahren nach Anspruch 1, ferner umfassend ein Auswählen eines Transplantationstyps durch einen Arzt und ein Bestimmen des mindestens einen geometrischen Merkmals, wobei das mindestens eine geometrische Merkmal als Funktion des ausgewählten Transplantationstyps bestimmt wird.

3. Verfahren nach Anspruch 2, wobei der Wert des mindestens einen geometrischen Merkmals ferner als eine Funktion des gewählten Transplantationstyps geschätzt wird.

4. Verfahren nach einem der Ansprüche 1 bis 3, ferner umfassend ein Auswählen mindestens eines virtuellen 3D-Basisobjekts aus einer Vielzahl von virtuellen 3D-Basisobjekten, wobei die Vielzahl von virtuellen 3D-Basisobjekten virtuelle 3D-Basisobjekte unterschiedlicher Größe und/oder unterschiedlicher Formen umfasst und das virtuelle 3D-Objekt das mindestens eine ausgewählte virtuelle 3D-Basisobjekt umfasst.

5. Verfahren nach Anspruch 4, wobei das mindestens eine ausgewählte virtuelle 3D-Basisobjekt die Form einer Olive, eines Parallelepipeds, eines Kegels oder einer Kombination davon hat.

6. Verfahren nach einem der Ansprüche 4 oder 5, bei dem mehrere virtuelle 3D-Basisobjekte ausgewählt werden, die einen Satz virtueller 3D-Basisobjekte bilden, wobei das virtuelle 3D-Objekt aus der Kombination der virtuellen 3D-Basisobjekte des Satzes virtueller 3D-Basisobjekte resultiert.

7. Verfahren nach einem der Ansprüche 4 bis 6, ferner umfassend eine homothetische Anpassung der Größe des mindestens einen ausgewählten virtuellen 3D-Basisobjekts oder eine homothetische Anpassung der Größe des virtuellen 3D-Objekts.

8. Verfahren nach einem der Ansprüche 1 bis 7, wobei die Darstellung des virtuellen 3D-Objekts eine Vielzahl von Punkten umfasst, die sich auf einer äußeren Fläche des virtuellen 3D-Objekts befinden, wobei ein Modifizieren der Darstellung des virtuellen 3D-Objekts ein Auswählen eines Punktes aus der Vielzahl von Punkten und ein Bewegen des ausgewählten Punktes umfasst, wobei das Bewegen des ausgewählten Punktes eine entsprechende Verformung des virtuellen 3D-Objekts bewirkt.

9. Verfahren nach Anspruch 8, wobei der ausgewählte Punkt in einem 2D-Bild ausgewählt wird, das einen Querschnitt durch das 3D-Volumen darstellt.

10. Verfahren nach einem der Ansprüche 1 bis 9, ferner umfassend ein Auswählen eines standardisierten virtuellen 3D-Objekts aus einer Vielzahl von standardisierten virtuellen 3D-Objekten als eine Funktion einer Größe und einer Form des virtuellen 3D-Objekts, wobei das Auswählen eines standardisierten virtuellen 3D-Objekts bei einer Änderung der Darstellung des virtuellen 3D-Objekts wiederholt wird.

11. Verfahren nach Anspruch 10, ferner umfassend ein Bereitstellen von Knochentransplantatmaterial, das dem ausgewählten standardisierten virtuellen 3D-Objekt entspricht, und ein Fräsen des bereitgestellten Knochentransplantatmaterials entsprechend dem virtuellen 3D-Objekt.

12. Verfahren nach einem der Ansprüche 1 bis 10, ferner umfassend ein Erzeugen eines 3D-Modells des virtuellen 3D-Objekts, wobei das erzeugte 3D-Modell ein 3D-Drucken eines entsprechenden Knochentransplantatmaterials oder ein Fräsen von Knochentransplantatmaterial entsprechend dem virtuellen 3D-Objekt ermöglicht.

13. Computerprogrammprodukt für eine programmierbare Vorrichtung, wobei das Computerprogrammprodukt Anweisungen zum Ausführen jedes Schritts des Verfahrens nach einem der Ansprüche 1 bis 12 enthält, wenn das Programm geladen und von einer programmierbaren Vorrichtung ausgeführt wird.

14. Vorrichtung zum dynamischen Schätzen von Merkmalen eines benötigten dentalen Knochentransplantationsmaterials, wobei die Vorrichtung einen Mikroprozessor umfasst, der zur Ausführung jedes der Schritte des Verfahrens nach einem der Ansprüche 1 bis 12 konfiguriert ist.

## Revendications

1. Procédé informatique d'aide dynamique d'un praticien dans la préparation d'une opération de greffe osseuse dentaire, le procédé comprenant les étapes consistant à :
- fournir au moins une image représentant un volume 3D d'au moins une partie d'un élément de mâchoire où une greffe osseuse doit être effectuée ;
- ajouter, à la au moins une image, une représentation d'un objet virtuel 3D représentant un matériau de greffe osseuse dentaire (110, 125, 145, 160, 170) ;
- estimer une valeur d'au moins une caractéristique géométrique relative au matériau de greffe osseuse à utiliser, en fonction de caractéristiques géométriques de l'objet virtuel 3D représenté, les caractéristiques géométriques de l'objet virtuel 3D représenté comprenant au moins l'un parmi une forme, un volume et une taille ;
- permettre à un utilisateur de modifier la représentation de l'objet virtuel 3D ; et
- lors de la modification de la représentation de l'objet virtuel 3D, mettre à jour la valeur estimée de la au moins une caractéristique géométrique, selon la modification de la représentation de l'objet virtuel 3D,
la valeur estimée aidant le praticien à déterminer le matériau de greffe osseuse dentaire (110, 125, 145, 160, 170) nécessaire.

2. Procédé selon la revendication 1, comprenant en outre l'étape consistant à sélectionner un type de greffe, par un praticien, et à déterminer la au moins une caractéristique géométrique, la au moins une caractéristique géométrique étant déterminée en fonction du type de greffe sélectionné.

3. Procédé selon la revendication 2, dans lequel la valeur de la au moins une caractéristique géométrique est en outre estimée en fonction du type de greffe sélectionné.

4. Procédé selon l'une quelconque des revendications 1 à 3, comprenant en outre l'étape consistant à sélectionner au moins un objet virtuel 3D de base dans une pluralité d'objets virtuels 3D de base, la pluralité d'objets virtuels 3D de base comprenant des objets virtuels 3D de base de différentes tailles et/ou de différentes formes, l'objet virtuel 3D comprenant le au moins un objet virtuel 3D de base sélectionné.

5. Procédé selon la revendication 4, dans lequel le au moins un objet virtuel 3D de base sélectionné a la forme d'une olive, d'un parallélépipède, d'un cône ou d'une combinaison de ceux-ci.

6. Procédé selon la revendication 4 ou la revendication 5, comprenant la sélection de plusieurs objets virtuels 3D de base formant un ensemble d'objets virtuels 3D de base, l'objet virtuel 3D résultant de la combinaison des objets virtuels 3D de base de l'ensemble d'objets virtuels 3D de base.

7. Procédé selon l'une quelconque des revendications 4 à 6, comprenant en outre l'étape consistant à ajuster homothétiquement la taille d'au moins un du au moins un objet virtuel 3D de base sélectionné ou à ajuster homothétiquement la taille de l'objet virtuel 3D.

8. Procédé selon l'une quelconque des revendications 1 à 7, dans lequel la représentation de l'objet virtuel 3D comprend une pluralité de points situés sur une surface externe de l'objet virtuel 3D, la modification de la représentation de l'objet virtuel 3D comprenant la sélection d'un point de la pluralité de points et le déplacement du point sélectionné, le déplacement du point sélectionné provoquant la déformation de l'objet virtuel 3D en conséquence.

9. Procédé selon la revendication 8, dans lequel le point sélectionné est sélectionné dans une image 2D représentant une section du volume 3D.

10. Procédé selon l'une quelconque des revendications 1 à 9, comprenant en outre l'étape consistant à sélectionner un objet virtuel 3D normalisé dans une pluralité d'objets virtuels 3D normalisés en fonction d'une taille et d'une forme de l'objet virtuel 3D, la sélection d'un objet virtuel 3D normalisé étant répétée lors de la modification de la représentation de l'objet virtuel 3D.

11. Procédé selon la revendication 10, comprenant en outre l'étape consistant à fournir un matériau de greffe osseuse correspondant à l'objet virtuel 3D normalisé sélectionné et à fraiser le matériau de greffe osseuse fourni selon l'objet virtuel 3D.

12. Procédé selon l'une quelconque des revendications 1 à 10, comprenant en outre l'étape consistant à générer un modèle 3D de l'objet virtuel 3D, le modèle 3D généré rendant possible l'impression 3D d'un matériau de greffe osseuse correspondant ou le fraisage du matériau de greffe osseuse selon l'objet virtuel 3D.

13. Produit de programme informatique pour un appareil programmable, le produit de programme informatique comprenant des instructions pour effectuer chaque étape du procédé selon l'une quelconque des revendications 1 à 12 lorsque le programme est chargé et exécuté par un appareil programmable.

14. Dispositif d'estimation dynamique des caractéristiques d'un matériau de greffe osseuse dentaire nécessaire, le dispositif comprenant un microprocesseur configuré pour effectuer chacune des étapes du procédé selon l'une quelconque des revendications 1 à 12.
